# EUROPÄISCHE PATENTANMELDUNG

(11) **EP 1 764 005 A1**
(43) Veröffentlichungstag der Anmeldung: **21.03.2007**
(21) Anmeldenummer: 06116389.5
(22) Anmeldetag: 30.06.2006
(51) Int. Cl.: A23L 3/16, A23L 3/18, B65B 55/02, A23L 3/04, A61L 2/04

(54) **Vorrichtung und Verfahren zum Durchführen von Wärmebehandlungen**

(30) Priorität: 27.07.2005 DE 102005035018
(71) Anmelder: Hoegger AG, 9230 Flawil (CH)
(72) Erfinder: Treier, René, 9242, Oberuzwil (CH)
(74) Vertreter: Büchel, von Révy & Partner

(57) **Zusammenfassung**

Zum Durchführen von Wärmebehandlungen mit Behandlungszonen, von denen min-destens eine als Heizzone (15) und mindestens eine als Kühlzone (20) ausgebildet ist, werden Produkte (2) von einem Eintrittsbereich durch die Behandlungszonen zu einem Austrittsbereich gefördert. Eine Behandlungszone ist als Rekuperationszone (10) aus-gebildet, wobei ein Transfermedium in der Rekuperationszone (10) einen Wärmeaus-tausch zwischen unterschiedlich warmen Produkten (2) erzielbar macht. Eine Förder-vorrichtung (4) führt durch die Rekuperationszone (10), anschliessend durch die min-destens eine Heizzone (15), nach der mindestens einen Heizzone (15) nochmals durch die Rekuperationszone (10) und anschliessend durch die mindestens eine Kühlzone (20). Im Betrieb der Vorrichtung (1) werden die Produkte (2) in der Rekuperations-zone (10) erwärmt, in der mindestens einen Heizzone (15) weiter mit Wärme be-handelt, nach der mindestens einen Heizzone (15) erneut in die Rekuperationszone (10) geführt, dort gekühlt und anschliessend in der mindestens einen Kühlzone (20) weiter gekühlt. Durch das Zusammenführen der behandelten und unbehandelten Produkte in der Rekuperationszone (10) mit einem Übertragungsmedium kann mit kleinem Aufwand eine äusserst effiziente Erhöhung der Energieeffizienz erzielt werden.

## Beschreibung

Die Erfindung bezieht sich auf Vorrichtungen nach dem Oberbegriff des Anspruches 1 und auf Verfahren nach dem Oberbegriff des Anspruches 9.

Bei der Behandlung oder der Herstellung von Produkten werden häufig Wärmebehand-lungsschritte mit kontinuierlich arbeitenden Vorrichtungen durchgeführt. Die Produkte werden dabei entlang eines Förderweges in Behandlungszonen aufgeheizt und an-schliessend wieder abgekühlt. Wärmebehandlungen sind insbesondere zur Behandlung von Nahrungsmitteln wichtig. Zur Wärmeübertragung wird eine Flüssigkeit, Dampf oder ein Gas verwendet. Die Wahl des Wärme-Transfermediums hängt von der gewünsch-ten Temperaturänderung ab. Bei Wärmebehandlungen mit Temperaturen unter 100°C, also beispielsweise bei der Pasteurisierung, kann Wasser, oder gegebenenfalls Wasser mit Zusätzen, verwendet werden. Zum Erwärmen wird in einer Heizzone zumindest ein erster Kreislauf für ein erstes Medium ausgebildet, wobei mit einer Heizvorrichtung Wärme in diesen ersten Kreislauf eingebracht wird, welche vom ersten Medium zumin-dest teilweise an das Produkt abgegeben wird. Zum Abkühlen wird in einer Kühlzone zumindest ein zweiter Kreislauf für ein zweites Medium ausgebildet, wobei das zweite Medium vom Produkt erwärmt wird und eine Kühlvorrichtung Wärme aus diesem zwei-ten Kreislauf entnimmt. Weil bei vielen gängigen Temperaturbehandlungen grosse Temperaturänderungen, beispielsweise über 50°C, insbesondere im Bereich von 70°C bis 90°C, erzielt werden, sind sehr hohe Heizleistungen und Kühlleistungen gefragt. Bei diesen grossen Energieumsätzen können effizientere Verfahren gegenüber den be-kannten Verfahren grosse Einsparungen erzielen.

Aus der DE 30 29 113 A1 ist eine Tunnel-Pasteurisieranlage bekannt, welche mehrere Aufwärmzonen, mehrere Kühlzonen und zwischen den Aufwärm- und den Kühlzonen eine Pasteurisierzone umfasst. Den Aufwärm- und Abkühlzonen sind Leitungsverbin-dungen zugeordnet, um Flüssigkeit, die in einer Kühlzone vom Produkt erwärmt wurde, einer Aufwärmzone zuzuführen, wo sie die Wärme an das Produkt abgeben kann. Zur gezielten Prozessführung müssen nebst den Verbindungsleitungen auch Pumpen und Steuereinrichtungen mit Temperaturfühlern und Ventilen eingesetzt werden, welche eine gewünschte Temperaturführung des Produktes gewährleisten. Der Aufbau der Anlage und ihre Steuerung sind sehr kompliziert. Eine ähnliche Lösung ist auch aus dem Patent US 6 352 021 B1 bekannt. Bei den beschriebenen Anlagen muss eine be-trächtliche Menge Energie eingesetzt werden, um das Wasser zwischen den Zonen hin und her zu pumpen. Daher ist die durch die Rekuperation gesamthaft erzielbare Einspa-rung nicht genügend hoch.

Das Patent DE 195 80 782 T1 beschreibt eine Vorrichtung zum Pasteurisieren mit Wärmerekuperation, bei welcher der Aufwand für den Austausch des Wassers zwi-schen verschiedenen Zonen reduziert ist. Dazu wird ein Paar von Fördereinrichtun-gen gleicher Länge in entgegengesetzter Richtung nebeneinander aufgebaut. Ab-kühlzonen der ersten Fördereinrichtung liegen direkt über Erwärmungszonen der zweiten Fördereinrichtung und entsprechend kann ein Austausch von Wasser zwi-schen diesen beieinander liegenden Zonen mit kleinem Aufwand erzielt werden. Kühlwasser, das vom Produkt in der Kühlzone erwärmt wurde, kann diese Wärme an das Produkt in der darunter liegenden Erwärmungszone abgeben. Analog wird auch vom Produkt gekühltes Heizwasser der ersten Fördereinrichtung zum Kühlen von heissem Produkt in der zweiten Fördereinrichtung eingesetzt. Die bei dieser Rekuperation entstehenden Temperaturunterschiede sind klein und entsprechend ist auch die Wärmeübertragung minimal bzw. die Temperaturänderung eines Pro-duktes klein. Weil die Temperaturänderungen auch langsam erfolgen, muss eine grosse Zonenlänge oder eine kleine Fördergeschwindigkeit vorgesehen werden, was zu grossen Vorrichtungen bzw. kleinen Durchsätzen führt.

Die beiden Fördereinrichtungen, bzw. die daran angeordneten Erwärmungs- und Kühlzonen müssen symmetrisch aufgebaut werden, damit zusammenpassende Zo-nen beieinander liegen. Es gibt nun aber viele Temperaturbehandlungen bei denen ein symmetrischer Temperaturverlauf, bzw. ein gleichartiges Anheben und Absen-ken der Temperatur nicht zweckmässig ist. Gerade beim Pasteurisieren kann es wichtig sein, dass das Produkt vor der Pasteurisierungszone langsam erwärmt, nach der Pasteurisierungszone aber schnell abgekühlt wird.

Für asymmetrische Temperaturverläufe ist die Lösung gemäss der DE 195 80 782 T1 nicht vorteilhaft einsetzbar. Zudem hat eine Anlage gemäss der DE 195 80 782 T1 aufgrund der beiden benötigten Fördereinrichtungen einen komplizierten Aufbau. Die Aufteilung des Produkteflusses in zwei Teilflüsse und das abschliessende Zu-sammenführen der beiden Teilflüsse ist mit zusätzlichem Förderaufwand verbun-den. Ein weiterer Nachteil der beiden gegenläufig betriebenen Fördereinrichtungen besteht darin, dass sie sich über grosse Längen erstrecken, was mit einem grossen Platzbedarf im Produktionsbetrieb verbunden ist.

Aus der Schrift EP 474 526 A1 ist eine Sterilisationsvorrichtung bekannt, welche die Produkte nach dem Durchlauf einer vertikalen hydrostatischen Behandlungszone in Aufnahmekörben mit Ketten durch übereinander angeordnete horizontale Behand-lungsabschnitte führt. Unten befindet sich eine Sterilisationszone mit mehreren übereinander angeordneten Abschnitten. Die Temperatur wird mit Heissdampf auf 125°C gehalten. Direkt über der Sterilisationszone ist eine Vorwärmzone mit über-einander angeordneten Abschnitten ausgebildet. Die aus der Sterilisationszone auf-steigende Abwärme gelangt in die Vorwärmzone. Über der Vorwärmzone befindet sich eine Kühlzone mit mehreren übereinander angeordneten Abschnitten. Der Platzbedarf dieser Anlage ist reduziert. Die Energie wird aber nicht effizient ver-wendet.

Die erfindungsgemässe Aufgabe besteht nun darin eine Vorrichtung und ein Verfahren zu finden, bei denen Energie effi-zient eingesetzt wird und welche einfach aufgebaut sind. Die Wärmebehandlung sollte an unterschiedliche, insbesondere auch asymmetri-sche, Temperaturverläufe angepasst werden können. Vorzugsweise soll auch der für die Vorrichtung benötigte Platz klein sein.

Die Aufgabe wird durch die Merkmale der Ansprüche 1 und 9 gelöst. Die abhängigen Patentansprüche beschreiben bevorzugte Ausführungsformen.

Im Rahmen der Erfindung wurde erkannt, dass die Energieeffizienz dann besonders hoch ist, wenn die Wärmeenergie des Produktes am Ende der Wärmebehandlung mög-lichst gut für das Vorwärmen des Produktes eingesetzt werden kann. In einer Rekupe-rationszone kann Wärme des Produktes direkt an noch nicht behandelte Produkte übertragen werden. Als Übertragungsmedium wird eine Flüssigkeit, vorzugsweise Was-ser, eingesetzt. Die Produkte werden also nach der Hauptbehandlung, insbesondere der Pasteurisierung, oder gegebenenfalls der Sterilisierung, mit einer hohen Tempera-tur in die Rekuperationszone geführt. Gleichzeitig werden auch noch nicht erwärmte Produkte mit einer tiefen Temperatur in die Rekuperationszone geführt. Das Übertra-gungsmedium der Rekuperationszone ermöglicht eine Wärmeübertragung von den be-handelten, heissen Produkten zu den unbehandelten, kühlen Produkten. Im kontinuierli-chen Betrieb stellt sich im Übertragungsmedium der Rekuperationszone eine mittlere Temperatur ein, die zwischen der Temperatur der zusammengeführten heissen und kühlen Produkte liegt. Entsprechend geben die heissen Produkte Wärme an das Wär-meübertragungsmedium ab und die kühlen Produkte nehmen Wärme auf, so dass beim Austritt aus der Rekuperationszone die behandelten Produkte etwas gekühlt und die noch nicht behandelten Produkte etwas erwärmt sind.

Nach dem Verlassen der Rekuperationszone werden die behandelten und etwas gekühlten Produkte in eine Kühlzone weiter geführt, wo sie von einem Kühlmedium auf die gewünschte Austrittstemperatur weiter gekühlt werden. Die noch nicht be-handelten etwas erwärmten Produkte werden in eine Heizzone gebracht, wo sie von einem Heizmedium auf die gewünschte Behandlungstemperatur gebracht und für eine benötigte Zeit auf dieser Temperatur gehalten werden.

Durch das Zusammenführen der behandelten und unbehandelten Produkte in einer Rekuperationszone mit einem Übertragungsmedium kann mit kleinem Aufwand eine äusserst effiziente Erhöhung der Energieeffizienz erzielt werden. Es braucht nicht zwei vollständige parallel angeordnete Fördereinrichtungen, wie sie in der DE 195 80 782 T1 beschrieben sind, sondern lediglich eine erste Zu- und Weiterführung für die behandelten sowie eine zweite Zu- und Weiterführung für die unbehandelten Produkte. Der erfindungsgemässe Rekuperationsschritt in einer speziellen Rekupe-rationszone erzielt einen Wärmeaustausch zwischen behandelten Produkten, die beim Eintritt in die Rekuperationszone im Wesentlichen die maximale Temperatur der Temperaturbehandlung aufweisen, und zwischen Produkten, welche noch nicht in der Heizzone waren. Die grossen Temperaturunterschiede zwischen unterschied-lich warmen Produkten in der Rekuperationszone ermöglichen einen schnellen Wärmeaustausch und entsprechend schnelle Temperaturänderungen. Nicht sym-metrische Temperaturverläufe können durch die unterschiedliche Auslegung der Heiz- und der Kühlzonen er möglicht werden.

Der Energieaustausch in der Rekuperationszone kann in einem Flüssigkeitsbad durchgeführt werden, vorzugsweise aber wird der Austausch durch das Berieseln der Produkte mit dem flüssigen Übertragungsmedium erzielt, wobei die Flüssigkeit über die heissen und über die kalten Produkte rieselt und eine Rekuperationszirku-lation das flüssige Übertragungsmedium in einem Kreislauf bewegt. Beim Berieseln wird vorzugsweise ein grosser Wasserdurchsatz vorgesehen. Zweckmässig ist es, wenn der Wasserdurchsatz 20 bis 70, insbesondere aber 50, mal grösser ist als der Produktedurchsatz. Das Flüssigkeits-bad oder die bevorzugte Rekuperationszirkulation ist einfach aufgebaut. Es werden keine komplizierten Leitungsverbindungen zwischen räumlich getrennten Zonen benö-tigt. Auch auf komplizierte Steuereinrichtungen mit Temperaturfühlern und Ventilen kann verzichtet werden, weil der Wärmeaustausch zwi-schen verschieden heissen Pro-dukten in einer gemeinsamen Zone erfolgt. Das Zu-sammenführen der verschieden heissen Produkte kann mit einfachen Mitteln erfolgen, so dass die gesamthaft erziel-bare Energieeinsparung recht hoch ist.

Der Platzbedarf der Vorrichtung ist klein, wenn zumindest die Rekuperationszone über-einander liegende horizontale Behandlungsabschnitte umfasst. Einfache Transportwege ergeben sich, wenn die kalten Produkte von einer ersten Seite her in die Rekuperati-onszone ein- und austreten. Entsprechend können die heissen Produkte von einer ge-genüberliegenden Seite her ein- und austreten. Die heissen und die kalten Produkte bewegen sich vorzugsweise je zweimal auf übereinander liegenden Behandlungsab-schnitten durch die Rekuperationszone. Es versteht sich von selbst, dass es sich dabei jeweils auch um ein Vielfaches von zwei handeln kann, wobei die heissen und kalten Produkte bei angepassten Zu- und Weiterführungen ungleich viele Male durch die Re-kuperationszone bewegt werden können. Im Wärme-Transfermedium stellt sich bei kon-stantem Betrieb eine im Wesentlichen konstante Rekuperationstemperatur ein. Durch eine richtige Auswahl und Dimensionierung der Behandlungsabschnitte der Rekupera-tionszone lässt sich eine namhafte Energieeinsparung erzielen. Die benötigte Heizleis-tung der Heizzone und die benötigte Kühlleistung der Kühlzone können wesentlich re-duziert werden.

Bei einer bevorzugten Ausführungsform umfasst auch die Kühlzone und/oder die Heizzone übereinander liegende horizontale Behandlungsabschnitte. Einfache Trans-portwege ergeben sich, wenn die Produkte von der ersten Seite her in die Heizzone eintreten und gegen die zweite Seite hin austreten. In die Kühlzone erfolgt der Eintritt vorzugsweise von der zweiten Seite her. Die Anzahl der horizontalen Behandlungsab-schnitte in der Kühlzone und in der Heizzone wird an die gewünschte Kühl- bzw. Heiz-wirkung angepasst.

Unterschiedliche Rekuperationseffekte und unterschiedliche Kühl- oder Heizleistungen können durch das Variieren der Anzahl von horizontalen Behandlungsabschnitten in der jeweiligen Zone erzielt werden, ohne dass sich dabei die horizontale Ausdehnung der Vorrichtung ändert.

Die Produkte werden in einer erfinderischen Vorrichtung zuerst durch die Rekupera-tionszone geführt, wo sie vorgewärmt werden. Anschliessend gehen die Produkte durch die Heizzone, wo sie auf die gewünschte Temperatur erwärmt und während einer ge-wünschten Zeit auf dieser Temperatur gehalten werden. Nach dem Austritt aus der Heizzone werden die Produkte wieder durch die Rekuperationszone geführt, um Wärme an die unbehandelten Produkte abzugeben. Die gekühlten Produkte gelangen von der Rekuperationszone in die Kühlzone, wo sie auf die Abgabetemperatur abgekühlt wer-den. Es versteht sich von selbst, dass auch zwei oder mehr Heizzonen und/oder Kühl-zonen ausgebildet werden können.

Die Fördervorrichtung, welche die Produkte durch die verschiedenen Behandlungszo-nen und auch zwischen den Zonen fördert, kann Tablett- bzw. Tray-Förderer und/oder Bandförderer umfassen. Es ist grundsätzlich jede Art von Fördermittel einsetzbar, wenn das Wärme-Transfermedium den nötigen Kontakt zu den Produkten machen kann. Wenn die Produkte berieselt werden, so ist es zweckmässig, dass das Wärme-Trans-fermedium durch die Auflagefläche des jeweiligen Förderers durchtreten kann. In einem Bad muss der Förderer gewährleisten, dass die Produkte kontinuierlich durch das Bad gefördert werden. Weil die Produkte häufig eine kleinere Dichte haben als die Flüssig-keit des Bades, muss gewährleistet werden, dass die im Bad nach oben strebenden Produkte nicht zu Verklemmungen oder Verstopfungen führen. Dazu können gegebe-nenfalls Niederhalteeinrichtungen eingesetzt werden. Bei Tabletts kann etwa ein am Tablett befestigter oberer Halterost und bei Bändern kann ein oberes Niederhalteband eingesetzt werden, wobei das Niederhalteband synchron mit dem Förderband bewegt wird.

Besonders vorteilhaft kann die erfindungsgemässe Lösung zum Pasteurisieren einge-setzt werden, weil beim Pasteurisieren keine Temperaturen über 100°C erzielt werden müssen und somit in der Kühlzone, in der Rekuperationszone sowie in der Heizzone Wasser als Transfermedium eingesetzt werden kann. Die Rekuperationszone umfasst eine im Wesentlichen geschlossene Zirkulation oder gegebenenfalls ein Bad. In der Heizzone muss das Transfermedium von einer Heizvorrichtung geheizt werden. Das Kühlwasser der Kühlzone wird gekühlt oder ersetzt. Beim Anfahren der Vorrichtung fällt die Erwärmung in der Rekuperationszone weg, oder das Transfermedium der Rekupe-rationszone muss auf eine gewünschte Temperatur erwärmt werden. Am Ende einer Produktebehandlungsphase muss das Transfermedium der Rekuperationszone gege-benenfalls gekühlt, bzw. teilweise ersetzt, werden, damit die behandelten Produkte in der Rekuperationszone genügend vorgekühlt werden.

Die erfindungsgemässe Lösung ist nicht auf die Behandlung von Lebensmitteln be-schränkt. Bevorzugt wird sie aber zum Pasteurisieren beispielsweise von verpackten Wurstwaren oder Frischteigwaren eingesetzt.

Die Zeichnungen erläutern die Erfindung anhand von zwei Aus-füh-rungsbeispielen, auf die sie aber nicht eingeschränkt ist. Dabei zeigt die
Fig. 1 eine schematische Darstellung eines vertikalen Schnittes durch einen Pasteur mit übereinander liegenden Behandlungsabschnitten, und
Fig. 2 eine schematische Darstellung eines Pasteurs mit einem Bad in der Rekupera-tionszone

Fig. 1 zeigt einen Pasteur 1 mit übereinander liegenden Behandlungsabschnitten. Die zu pasteurisierenden Produkte 2 werden von einer aus dem Stande der Technik be-kannten Beladestation 3 an eine Fördervorrichtung 4 des Pasteurs 1 übergeben. Bei der dargestellten Ausführungsform umfasst die Fördervorrichtung 4 zur Aufnahme der Produkte 2 Aufnahmetabletts 5, die in Förderrichtung hintereinander im wesentlichen horizontal ausgerichtet durch den Pasteur 1 bewegbar sind. Auf der Fig. 1 sind bei-spielhaft einzelne Tabletts 5 dargestellt. Zum Führen der Tabletts 5 umfasst die Förder-vorrichtung 4 Horizontalführungen 6 in den horizontalen Behandlungsabschnitten und teilweise dargestellte Vertikalführungen 7 zwischen aneinander anschliessenden verti-kal versetzten Behandlungsabschnitten. Die Führungen stellen sicher, dass die Tabletts 5 im Wesentlichen immer horizontal ausgerichtet sind. Zum Vorschieben der Tabletts 5 werden Antriebsmittel, vorzugsweise zwei parallel geführte Ketten 8 eingesetzt, die je auf einer Seite der Tabletts 5 geführt und mit den Tabletts 5 verbunden sind. Bei einer Entladestation 9 werden die behandelten Produkte 2 von den Tabletts 5 entladen. Die leeren Tabletts gelangen von der Entladestation 9 direkt zur Beladestation 3.

Die mit Produkten 2 beladenen Tabletts 5 gelangen von der Beladestation 3 direkt in eine Rekuperationszone 10. Die Fördervorrichtung 4 umfasst in der Rekuperationszone 10 zwei erste horizontalen Behandlungsabschnitte 11a und 11b. Die Tabletts 5 gelan-gen auf den unteren der beiden ersten Behandlungsabschnitte 11a über eine erste Zu-führung 17, bzw. einen Zuführungsbereich der Fördervorrichtung 4, von einer ersten Seite, bzw. von der Beladestation 3, her. Die beiden ersten Behandlungsabschnitte 11a, 11b sind auf einer zweiten Seite durch eine vertikale Übergabeeinrichtung miteinander verbunden. In der dargestellten Ausführungsform besteht die Übergabevorrichtung im Wesentlichen aus Umlenkrollen 13 für die Ketten 8 und aus nicht dargestellten Verti-kalführungen. Vom Ende des oberen ersten Behandlungsabschnittes 11 b gelangen die Tabletts 5 über eine erste Weiterführung 14 in eine Heizzone 15, welche drei überein-ander liegende Heiz-Behandlungsabschnitte 16 umfasst und vorzugsweise über der Rekuperationszone 10 liegt.

Die aus der Heizzone 15 austretenden Tabletts gelangen über eine zweite Zuführung 18 von der zweiten Seite her in den oberen zweiten Behandlungsabschnitt 12a der Re-kuperationszone 10. Auf der ersten Seite sind die beiden zweiten Behandlungsab-schnitte 12a, 12b durch eine vertikale Übergabeeinrichtung miteinander verbunden. Vom unteren, zweiten Behandlungsabschnitt 12b führt eine zweite Weiterführung 19 zu einer Kühlzone 20, welche fünf übereinander liegende Kühl-Behandlungsabschnitte 21 umfasst.

Der Wärmeaustausch in der Rekuperationszone erfolgt zwischen den Produkten in den ersten Behandlungsabschnitte 11a, 11b und den Produkten in den zweiten Behand-lungsabschnitte 12a, 12b. Für die Wärmeübertragung wird eine Flüssigkeit, vorzugs-weise Wasser, mit den Produkten in Kontakt gebracht. Die Flüssigkeit rieselt aus einer Berieselungsfläche 22 über Tabletts 5 mit unbehandelten Produkten in den ersten Be-handlungsabschnitten 11a, 11b und darunter über Tabletts 5 mit heissen Produkten, die aus der Heizzone 15 kommend durch die zweiten Behandlungsabschnitte 12a, 12b ge-fördert werden. Damit das Wasser nach dem Auftreffen auf Tabletts 5 weiterrieselt, ha-ben diese Durchtrittsöffnungen. Unter dem untersten Behandlungsabschnitt der Reku-perationszone ist eine Auffangwanne 23 angeordnet. Das in der Auffangwanne 23 ge-sammelte Wasser wird wieder der Berieslungsfläche 22 zugeführt. Es wird somit von nicht dargestellten Zirkulationsleitungen und einer Pumpe in Zirkulation gehalten.

Weil die zirkulierende Flüssigkeit sowohl mit den kalten als auch mit den heissen Pro-dukten in Kontakt kommt, stellt sich in der Flüssigkeit eine Betriebstemperatur zwischen der Temperatur der kalten und der Temperatur der heissen Produkte ein. Die kalten Produkte werden erwärmt und die heissen Produkte werden gekühlt und somit erzielt das Transfermedium bzw. die Flüssigkeit in der Rekuperationszone einen Wärmeaus-tausch zwischen unterschiedlich warmen Produkten. Aufgrund der Vorerwärmung bzw. der Vorkühlung in der Rekuperationszone ist die benötige Heiz- und Kühlleistung ge-genüber einem Verfahren ohne Rekuperation reduziert.

Die Betriebstemperatur hängt davon ab, wie viele kalte und heisse Produkte sich gleichzeitig in der Rekuperationszone befinden. Die Gesamtlängen der ersten und der zweiten Behandlungsabschnitte müssen so gewählt werden, dass sich eine Temperatur im gewünschten Bereich einstellt. Damit das Transfermedium der Rekuperationszone bereits am Anfang einer Betriebsphase im Wesentlichen die Betriebstemperatur auf-weist, kann der Zirkulation auch eine Heizvorrichtung zugeordnet werden. Die Heizvor-richtung kann das Transfermedium auf die zu erwartende Betriebstemperatur erwär-men. Während des Betriebs kann auf ein Kühlen oder Heizen verzichtet werden.

Die Anzahl der Behandlungsabschnitte in der Heizzone 15 und der Kühlzone 20 wird an die jeweilige Wärmebehandlung angepasst. Es wird zumindest ein Abschnitt, insbeson-dere eine ungerade Anzahl von Abschnitten, vorgesehen. Die Heizzone 15 liegt vor-zugsweise über der Rekuperationszone 10 und die Kühlzone 20 liegt unter der Rekupe-rationszone 10. Dadurch ist ein äusserst kompakter Aufbau der Vorrichtung 1 möglich. Das Gehäuse 1a umschliesst die Vorrichtung 1 zumindest teilweise.

Zur Erwärmung der Produkte in der Heizzone 15 wird vorzugsweise eine heisse Flüs-sigkeit, insbesondere Wasser, mit den Produkten in Kontakt gebracht. Die Flüssigkeit rieselt aus einer Berieselungsfläche 22 über Tabletts 5 mit vorgewärmten Produkten durch die Heiz-Behandlungsabschnitte 16. Unter dem untersten Heiz-Behandlungsab-schnitt 16 der Heizzone 15 ist eine Auffangwanne 23 angeordnet. Das in der Auffang-wanne 23 gesammelte, leicht abgekühlte Wasser wird von einer nicht dargestellten Heizvorrichtung erwärmt und wieder der Berieslungsfläche 22 zugeführt. Das Heizme-dium wird somit von nicht dargestellten Zirkulationsleitungen und einer Pumpe in Zirku-lation gehalten.

Es versteht sich von selbst, dass die Erwärmung auch mit Heissdampf oder mit Mikro-wellen durchgeführt werden kann.

Zum Kühlen der Produkte in der Kühlzone 20 wird vorzugsweise eine kalte Flüssigkeit, insbesondere Wasser, mit den Produkten in Kontakt gebracht. Die Flüssigkeit rieselt aus einer Berieselungsfläche 22 über Tabletts 5 mit vorgekühlten Produkten durch die Kühl-Behandlungsabschnitte 21. Unter dem untersten Kühl-Behandlungsabschnitt 21 der Kühlzone 20 ist eine Auffangwanne 23 angeordnet. Das in der Auffangwanne 23 gesammelte, leicht erwärmte Wasser wird von einer nicht dargestellten Kühlvorrichtung gekühlt und wieder der Berieslungsfläche 22 zugeführt. Das Kühlmedium wird somit von nicht dargestellten Zirkulationsleitungen und einer Pumpe in Zirkulation gehalten.

Es versteht sich von selbst, dass zur Förderung der Produkte auch andere aus dem Stande der Technik bekannte Fördervorrichtungen eingesetzt werden können. Anstelle von durchgehenden Ketten 8 können auch andere Mittel eingesetzt werden um die ko-ordinierte Vorschubsbewegung der Tabletts zu gewährleisten. Anstelle von Tabletts können gegebenenfalls auch Bänder eingesetzt werden. Bei vertikalen Übergabeberei-chen können Vertikalförderer angeordnet werden. Die Fördervorrichtung kann entspre-chend der zu behandelnden Produkte sehr unterschiedlich ausgelegt werden.

Fig. 2 zeigt eine Ausführungsform bei der die Heizzone 15 und die Kühlzone 20 im We-sentlichen gleich ausgebildet ist, wie in Fig. 1. Die Rekuperationszone 10 umfasst aber ein Bad 24. Damit die Produkte auch im Bad 24 auf den Tabletts 5 verbleiben, werden beispielsweise Abdeckgitter 5a nach dem Befüllen der Tabletts 5 an den Tabletts 5 festgesetzt. Entsprechend umfassen die Beladestation 3 und die Entladestation 9 eine Hebe- und Schliessvorrichtung zum Abheben und wieder Aufsetzen der Abdeckgitter 5a. Wenn nun die unbehandelten kalten Produkte und die aus der Heizzone 15 kom-menden heissen Produkte durch das Bad 24 geführt werden, erzielt das Bad 24 einen Wärmeaustausch zwischen den heissen und kalten Produkten. Im Betrieb stellt sich im Bad 24 eine Betriebstemperatur ein.

Die Fördervorrichtung 4 umfasst im Bad 24 mindestens einen ersten und mindestens einen zweiten horizontalen Behandlungsabschnitt 11, 12, wobei der mindestens eine erste Behandlungsabschnitt 11 in Förderrichtung vor und der mindestens eine zweite Behandlungsabschnitt 12 nach der mindestens einen Heizzone 15 angeordnet ist. Das Bad 24 befindet sich in einer Wanne 24a. Die erste Zuführung 17 und die zweite Zufüh-rung 18 umfassen je einen Absenkbereich. Die erste Weiterführung 14 und die zweite Weiterführung 19 umfassen entsprechend einen Hebebereich. Bei der Verwendung eines Bades 24 kann in der Rekuperationszone 10 auf Zirkulationsleitungen und eine Pumpe verzichtet werden. Um auch am Anfang einer Betriebsphase die erwartete Be-triebstemperatur einstellen zu können, umfasst das Bad 24 gegebenenfalls eine Heiz-vorrichtung.

Die erfinderische Lösung kann auch bei Vorrichtungen eingesetzt werden, welche die Produkte im Wesentlichen nur in einer horizontalen Behandlungsebene bewegen. Dabei wird die Rekuperationszone beispielsweise zwischen der Heizzone und der Kühlzone angeordnet. Die Produkte können ausgehend von einer Beladestation bei der Rekupe-rationszone durch die Rekuperationszone in die Heizzone gefördert werden. In oder nach der Heizzone muss die Föderrichtung um 180° gedreht werden, so dass die Pro-dukte nach der Heizzone wieder zur Rekuperationszone gelangen. Beim zweiten Durchlaufen der Rekuperationszone werden die Produkte gegen die Kühlzone bewegt, welche sie nach dem Verlassen der Rekuperationszone durchlaufen.

## Patentansprüche

1. Vorrichtung (1) zum Durchführen von Wärmebehandlungen mit Behandlungszonen, von denen mindestens eine als Heizzone (15) und mindestens eine als Kühlzone (20) ausgebildet ist, und mit einer Fördervorrichtung (4) zum Fördern von Produk-ten (2) von einem Eintrittsbereich durch die Behandlungszonen zu einem Austritts-bereich, **dadurch** ge-kenn-zeichnet, dass eine Behandlungszone als Rekupera-tionszone (10) ausgebildet ist, wobei ein Transfermedium in der Rekuperationszone (10) einen Wärmeaustausch zwischen unterschiedlich warmen Produkten (2) er-zielbar macht, und die Fördervorrichtung (4) durch die Rekuperationszone (10), anschliessend durch die mindestens eine Heizzone (15), nach der mindestens einen Heizzone (15) nochmals durch die Rekuperationszone (10) und anschlies-send durch die mindestens eine Kühlzone (20) führt und somit im Betrieb der Vorrichtung (1) die Produkte (2) in der Rekuperationszone (10) erwärmt werden, in der mindestens einen Heizzone (15) weiter mit Wärme behandelt werden, nach der mindestens einen Heizzone (15) erneut in die Rekuperationszone (10) gelangen, dort gekühlt werden und anschliessend in der mindestens einen Kühl-zone (20) weiter gekühlt werden.

2. Vorrichtung (1) nach Anspruch 1, **dadurch gekennzeichnet, dass** die Fördervorrich-tung (4) in der Rekuperationszone (10) mindestens einen ersten und mindestens einen zweiten horizontalen Behandlungsabschnitt (11a, 11 b, 12a, 12b) umfasst, wobei der mindestens eine erste Behandlungsabschnitt (11a, 11b) in Förderrich-tung vor und der mindestens eine zweite Behandlungsabschnitt (12a, 12b) nach der mindestens einen Heizzone (15) angeordnet ist, die mindestens zwei Behandlungs-abschnitte (11a, 11 b, 12a, 12b) übereinander liegen und eine Zirkulationsvorrich-tung mit einer Berieselungsvorrichtung (22) das Transfermedium in der Form einer Flüssigkeit, vorzugsweise Wasser, in Zirkulation versetzen kann, wobei die Zirkula-tion über die Produkte (2) auf den Behandlungsabschnitten (11a, 11b, 12a, 12b) der Rekuperationszone (10) führt.

3. Vorrichtung (1) nach Anspruch 2, **dadurch gekennzeichnet, dass** zwei erste aneinan-der anschliessende Behandlungsabschnitte (11a, 11b) und zwei zweite an-einander anschliessende Behandlungsabschnitte (12a, 12b) in der Rekuperations-zone (10) angeordnet sind, wobei der untere der beiden ersten Behandlungsab-schnitte (11a) über eine erste Zuführung (17) von einer ersten Seite her mit Pro-dukten (2) gespeist wird, die beiden ersten Behandlungsabschnitt (11a, 11b) auf einer zweiten Seite durch eine vertikale Übergabeeinrichtung verbunden sind, der obere der beiden ersten Behandlungsabschnitte (11 b) über eine erste Weiterfüh-rung (14) mit der mindestens einen Heizzone (15) verbunden ist, der obere der bei-den zweiten Behandlungsabschnitte (12a) über eine zweite Zuführung (18) von der zweiten Seite her mit Produkten gespeist wird, die beiden zweiten Behandlungsab-schnitte (12a, 12b) auf einer ersten Seite durch eine vertikale Übergabeeinrichtung verbunden sind und der untere der beiden zweiten Behandlungsabschnitte (12b) über eine zweite Weiterführung (19) mit der mindestens einen Kühlzone (20) ver-bunden ist.

4. Vorrichtung (1) nach Anspruch 1, **dadurch gekennzeichnet, dass** die Fördervorrich-tung (4) in der Rekuperationszone (10) mindestens einen ersten und mindestens einen zweiten horizontalen Behandlungsabschnitt (11, 12) umfasst, wobei der min-destens eine erste Behandlungsabschnitt (11) in Förderrichtung vor und der min-destens eine zweite Behandlungsabschnitt (12) nach der mindestens einen Heiz-zone (15) angeordnet ist, die mindestens zwei Behandlungsabschnitte (11, 12) durch ein gemeinsames Bad (24) führen, in dem das Transfermedium in der Form einer Flüssigkeit, vorzugsweise Wasser, aufgenommen ist.

5. Vorrichtung (1) nach einem der Ansprüche 1 bis 4, **dadurch gekennzeichnet, dass** die Fördervorrichtung (4) in der mindestens einen Heizzone (15) mindestens einen horizontalen Heiz-Behandlungsabschnitt (16), vorzugsweise eine ungerade Anzahl von übereinander liegenden horizontalen Heiz-Behandlungsabschnitten (16), um-fasst und die mindestens eine Heizzone (15) über der Rekuperationszone (10) an-geordnet ist.

6. Vorrichtung (1) nach einem der Ansprüche 1 bis 5, **dadurch gekennzeichnet, dass** die Fördervorrichtung (4) in der mindestens einen Kühlzone (20) mindestens einen horizontalen Kühl-Behandlungsabschnitt (21), vorzugsweise eine gerade Anzahl von übereinander liegenden horizontalen Kühl-Behandlungsabschnitten (21), um-fasst und die mindestens eine Kühlzone (20) unter der Rekuperationszone (10) an-geordnet ist.

7. Vorrichtung (1) nach einem der Ansprüche 1 bis 6, **dadurch gekennzeichnet, dass** die Fördervorrichtung (4) zur Aufnahme der Produkte Aufnahmetabletts (5) um-fasst, die in Förderrichtung hintereinander im wesentlichen horizontal ausgerichtet durch die Vorrichtung (1) bewegbar sind, wobei sie bei einer Beladestation (3) mit Produkten (2) beladen und bei einer Entladestation (9) entladen sowie von der Entladestation (9) ohne Produkte (2) direkt zur Beladestation (3) gefördert werden.

8. Vorrichtung (1) nach Anspruch 7, **dadurch gekennzeichnet, dass** die Fördervorrich-tung (4) zum Führen der Tabletts (5) Horizontalführungen (6) in den horizontalen Behandlungsabschnitten (11a, 11b, 12a, 12b, 11, 12) und Vertikalführungen (7) zwischen aneinander anschliessenden vertikal versetzten Behandlungsabschnitten (11a, 11b, 12a, 12b, 11, 12) sowie zum Vorschieben der Tabletts (5) Antriebsmittel, vorzugsweise zwei parallel geführte Ketten (8), die je auf einer Seite der Tabletts (5) geführt und mit den Tabletts (5) verbunden sind, umfasst.

9. Verfahren zum Durchführen von Wärmebehandlungen in Behandlungszonen, von denen mindestens eine als Heizzone (15) und mindestens eine als Kühlzone (20) ausgebildet ist, wobei die Produkte (2) von einem Eintrittsbereich durch die Be-handlungszonen zu einem Austrittsbereich gefördert werden, **dadurch** ge-kenn-zeichnet, dass in einer Rekuperationszone (10) ein Rekuperationsschritt durch geführt wird, bei dem ein Transfermedium einen Wärmeaustausch zwischen unter-schiedlich warmen Produkten (2) erzielt, und die Produkte (2) von einer Fördervor-richtung (4) durch die Rekuperationszone (10), anschliessend durch die mindes-tens eine Heizzone (15), nach der mindestens einen Heizzone (15) nochmals durch die Rekuperationszone (10) und anschliessend durch die mindestens eine Kühl-zone (20) geführt werden, wobei die Produkte (2) in der Rekuperationszone (10) erwärmt werden, in der mindestens einen Heizzone (15) weiter mit Wärme be-handelt werden, nach der mindestens einen Heizzone (15) erneut in die Rekupe-rationszone (10) gelangen, dort gekühlt werden und anschliessend in der min-destens einen Kühlzone (20) weiter gekühlt werden.

10. Verfahren nach Anspruch 9, **dadurch gekennzeichnet, dass** die Produkte (2) in der Rekuperationszone (10) zur Durchführung des Rekuperationsschrittes in Förder-richtung vor der mindestens einen Heizzone (15) entlang mindestens eines ersten Behandlungsabschnittes (11a, 11b) und in Förderrichtung nach der mindestens einen Heizzone (15) entlang mindestens eines zweiten Behandlungsabschnittes (12a, 12b) geführt werden, die mindestens zwei Behandlungsabschnitte (11a, 11 b, 12a, 12b) übereinander liegen und das Transfermedium in der Form einer Flüssig-keit, vorzugsweise Wasser, in Zirkulation versetzt wird, wobei die Zirkulation über die Produkte (2) auf den Behandlungsabschnitten (11a, 11b, 12a, 12b) der Rekupe-rationszone (10) führt.

11. Verfahren nach Anspruch 9, **dadurch gekennzeichnet, dass** die Produkte (2) in der Rekuperationszone (10) zur Durchführung des Rekuperationsschrittes in Förder-richtung vor der mindestens einen Heizzone (15) entlang mindestens eines ersten Behandlungsabschnittes (11a, 11 b) und in Förderrichtung nach der mindestens einen Heizzone (15) entlang mindestens eines zweiten Behandlungsabschnittes (12a, 12b) geführt werden, wobei die Produkte (2) in den mindestens zwei Be-handlungsabschnitten (11a, 11b, 12a, 12b) durch ein gemeinsames Bad (24) ge-führt werden, in dem das Transfermedium in der Form einer Flüssigkeit, vorzugs-weise Wasser, aufgenommen ist.

12. Verfahren nach einem der Ansprüche 9 bis 11, **dadurch gekennzeichnet, dass** die Produkte (2) auf im Wesentlichen horizontal ausgerichteten Aufnahmetabletts (5) in Förderrichtung hintereinander durch die Behandlungszonen bewegt werden, wobei sie bei einer Beladestation (3) auf die Tabletts (5) geladen und bei einer Entlade-station (9) entladen werden.
